# EUROPEAN PATENT APPLICATION

(11) **EP 4 342 503 A1**
(43) Date of publication of application: **27.03.2024**
(21) Application number: 22196524.7
(22) Date of filing: 20.09.2022
(51) Int. Cl.: A61M 1/06

(54) **MILK EXPRESSION DEVICE AND METHOD FOR OPERATING THE SAME**

(71) Applicant: Koninklijke Philips N.V., 5656 AG Eindhoven (NL)
(72) Inventor: BOURQUIN, Yannyk Parulian Julian, Eindhoven (NL); OUWELTJES, Okke, Eindhoven (NL); WIJNOLTZ, Anna Louise, Eindhoven (NL)
(74) Representative: Philips Intellectual Property & Standards

(57) **Abstract**

Provided is a device (100) for expressing milk from a mammalian breast (102). The device comprises a nipple-enclosing wall (104) for interfacing with a nipple-comprising region (106) of the breast to define a chamber (108) between the nipple-enclosing wall and the nipple-comprising region of the breast in which an underpressure is providable. The chamber is, for example, connectable to an underpressure generator for decreasing a pressure in the chamber. The underpressure may assist milk to be expressed from the breast and/or may assist in retaining the interface between the nipple-enclosing wall and the nipple-comprising region. The device comprises an infrasound source (112) for producing infrasound. The infrasound souce comprises a moveable member (114) whose movement produces infrasound. The moveable member has a first side (114A) facing and in fluid communication with the chamber, and a second side (114B) opposite the first side. The second side is outside the chamber. A fluid connection (116) is provided between the chamber and the second side. The fluid connection may provide pressure equalization between the chamber and the second side of the moveable member so that both the first side and the second side of the moveable member experience the underpressure. The fluid connection is nonetheless configured to control fluid communication between the chamber and the second side of the moveable member during the movement of the moveable member and thus the fluid connection assists to control, e.g. enhance, a varying pressure experienced by the breast that is caused by the movement of the moveable member.

## Description

### FIELD OF THE INVENTION

This invention relates to a milk expression device.

### BACKGROUND OF THE INVENTION

The best source of nutrition for a baby is human milk given by a breastfeeding mother. The World Health Organization recommends to breast feed for at least one year, preferably longer. However, mothers often go back to work after only several weeks or months. To provide the best nutrition to their babies, mothers express milk using a breast pump. The expressed milk can be stored and given to the baby at a later stage and/or by somebody else.

Typically, breast pumps have two operating modes, namely a stimulation mode and an extraction mode. During the stimulation mode, the milk ejection reflex is stimulated to occur by higher frequency pumping (around 2 Hz). Once milk comes out of the breast it is advised to switch to an extraction mode, in which lower frequency (typically around 1 Hz) and higher intensity pumping is employed for more effective milk extraction.

Recently, there is a trend towards wearable breast pumps. These devices generally have a smaller form factor and can fit in the bra of the user.

Additionally, passive milk collectors exist, which can be placed on the breast and often allow the user to apply a static underpressure to hold the device in place and help in extracting milk. These collectors are meant to be used when breastfeeding to collect milk from the non-breastfeeding breast. The stimulation provided by the infant generates a milk ejection reflex on both breasts, and a simple passive vacuum is often enough to extract (some) milk.

### SUMMARY OF THE INVENTION

The invention is defined by the claims.

According to examples in accordance with an aspect of the invention, there is provided a device for expressing milk from a mammalian breast, the device comprising: a nipple-enclosing wall for interfacing with a nipple-comprising region of the breast to define a chamber between the nipple-enclosing wall and the nipple-comprising region of the breast in which an underpressure is providable; an infrasound source for producing infrasound, the infrasound source comprising a moveable member whose movement produces infrasound, the moveable member having a first side facing and in fluid communication with the chamber and a second side opposite the first side, the second side being outside the chamber; and a fluid connection between the chamber and the second side, the fluid connection being configured to control fluid communication between the chamber and the second side during the movement of the moveable member.

The fluid connection may provide pressure equalization between the chamber and the second side of the moveable member so that both the first side and the second side of the moveable member experience the underpressure, e.g. a decreased pressure caused by operation of an underpressure generator connectable to the chamber.

This may reduce underpressure-related disruption of movement of the moveable member.

The fluid connection is nonetheless configured to control fluid communication between the chamber and the second side of the moveable member during the movement, e.g. reciprocating movement, of the moveable member so as to control, together with the infrasound source, the varying pressure experienced by the breast.

Thus, the infrasound source, e.g. an electroacoustic transducer, varies the pressure in the chamber in order to provide mechanical stimulation to the breast.

In some embodiments, the fluid connection comprises, or is in the form of, a channel fluidly connecting the chamber to the second side of the moveable member.

In such embodiments, the control of the fluid communication between the chamber and the second side of the moveable member may be at least partly determined by the dimensions of the channel.

In other words, the dimensions of the channel may be selected such that the movement, e.g. reciprocating movement, of the moveable member causes a varying pressure to be experienced by the breast.

For example, the dimensions of the channel can be tuned such that frequencies of pressure variation, for instance, 0.5 to 20 Hz, are blocked by the channel so that the pressure variation is experienced by the breast, and frequencies of <0.5 Hz are communicated between the chamber and the second side of the moveable member.

In some embodiments, the fluid connection is configured to operate as a fluid frequency-dependent resistive element configured to selectively restrict fluid passage between the chamber and the second side of the moveable member driven by a pressure variation at a frequency caused by the movement of the moveable member. Thus, the fluid frequency-dependent resistive element may enable the pressure variation caused by the movement of the moveable member to be experienced by the breast.

Such a frequency-dependent resistive element may operate due to frequency dependent thermo-viscous losses in the channel included in or defining the fluid connection.

In such embodiments, the frequency of the pressure variation caused by the movement of the moveable member may be higher than a frequency of pressure variation resulting from operation of the underpressure generator.

In some embodiments, the device comprises a further chamber, with the second side of the moveable member facing and in fluid communication with the second chamber.

In such embodiments, the channel and the further chamber may fluidly connect the chamber to the second side of the moveable member, with the fluid connection being configured to operate, together with a volume of the further chamber, as an acoustic filter that selectively blocks a frequency of pressure variation caused by the movement of the moveable member from being communicated from the chamber to the further chamber. Thus, the acoustic filter may enable the pressure variation caused by the movement of the moveable member to be experienced by the breast.

In such embodiments, the frequency of the pressure variation caused by the movement of the moveable member may be higher than a frequency of pressure variation resulting from operation of the underpressure generator.

Hence the acoustic filter may be a low pass filter that allows a lower frequency pressure variation provided by the underpressure generator to be communicated between the chamber and the further chamber but blocks the higher frequency of pressure variation caused by the movement of the moveable member from being communicated from the chamber to the further chamber.

The length, L, and area, A, of the channel may be determined by using the well-known Helmholtz resonance frequency, f, defined by f = c/(2^{∗}pi) ^{∗} sqrt(A/(V^{∗}L)), where c is the speed of sound and V is the volume of the further chamber.

The Helmholtz resonator assists to avoid pressure waves on each side of the infrasound source's moveable member, which are in anti-phase, coming together in the chamber and cancelling each other out, and further assists to minimize the influence of the decreased pressure, e.g. static vacuum, on the neutral position of the moveable member, e.g. transducer diaphragm/membrane.

The air inside the channel may act as a mass, and the air inside the volume of the further chamber may act as a spring, forming a mass-spring system. Above the resonance frequency of this mass-spring system, the air may not pass through the channel because of its inertia, while at lower frequencies the inertia may be relatively low, and air can pass through the channel.

Hence, the fluid connection may act as a low pass filter.

The optimal choice of Helmholtz frequency may coincide with the lowest frequency of the pressure variation caused by the movement of the moveable member to be experienced by the breast.

The above may, strictly speaking, apply for an ideal system, without damping.

In practice, considering volume, V, and length, L, the channel may be relatively narrow, for example having a diameter in the order of a tenth of a millimeter.

Such narrow channels may have a high amount of thermo-viscous losses (proportional to diameter^4) resulting in a significantly (e.g. up to about 50 times) lower resonance frequency, and thus may act as a more effective low pass filter.

By way of a non-limiting illustrative example/ideal case, the volume, V, of the further chamber is 35 mL, the channel has a length, L, of 5 mm and a diameter, d, of 0.4 mm, giving a Helmholtz resonance frequency of about 20 Hz. When viscous losses are taken into account the resonance frequency lowers to about 0.5 Hz, which is a realistic resonance frequency in the present context.

More generally, the channel may have a diameter, d, of 0.05 to 0.9 mm and/or the channel may have a length of 1 to 10 mm.

Alternatively or additionally, the frequency of pressure variation caused by the movement of the moveable member may be equal to or greater than 0.5 Hz, such as 0.5 Hz to 20 Hz.

Alternatively or additionally, the frequency of pressure variation resulting from operation of the underpressure generator may be less than 0.5 Hz.

In some embodiments, the device comprises a three-way coupling member for connecting the underpressure generator to the chamber and to the further chamber.

Such a three-way coupling member may assist to provide pressure equalization between the chamber and the second side of the moveable member so that both the first side and the second side of the moveable member experience the decreased pressure caused by operation of the underpressure generator.

In at least some embodiments, the infrasound source comprises a piston attached to the moveable member, with movement of the piston causing the movement, e.g. reciprocating movement, of the moveable member.

It is noted that a change in the underpressure in the chamber can cause an offset in the starting position of the piston of the infrasound souce in spite of the inclusion of the fluid connection.

Thus, a starting position of the piston may be adjustable based on the underpressure in the chamber.

In this manner, the piston may be adjusted to compensate for a change in the vacuum level during the milk expression. For example, a direct current may be applied, in the case of the infrasound source being an electroacoustic transducer, to remove an offset to the starting position caused by a change in the underpressure.

In some embodiments, the device comprises an air permeable covering for restricting liquid passage from the breast to the moveable member, e.g. to the first and/or second side of the moveable member.

The air permeable covering may assist to protect the infrasound source from being damaged by the expressed milk in the chamber.

Any suitable type of air permeable covering can be contemplated, such as an air permeable covering made from Gore-Tex^{®}.

In some embodiments, the varying pressure experienced by the breast caused by the movement, e.g. reciprocating movement, of the moveable member comprises a pressure waveform. Such a pressure waveform may assist to mechanically stimulate the breast, and hence promote expression of milk from the breast.

Any suitable shape of pressure waveform may be contemplated. In some embodiments, the pressure waveform is a sawtooth waveform, a sinusoidal waveform, or a square waveform.

In some embodiments, the pressure waveform is user-selectable. Thus, the user can choose whichever pressure waveform is perceived as being most effective for them in terms of promoting expression of milk from the breast.

In such embodiments, a user interface may be configured to permit the user to make a selection of the pressure waveform, e.g. a sawtooth waveform, a sinusoidal waveform, or a square waveform, with the infrasound source being controllable to provide the user-selected pressure waveform.

In some embodiments, the device comprises a valve for selectively releasing the expressed milk from the chamber to a milk collection volume.

Since it may desirable for the chamber's volume to be kept as small as possible in order to facilitate mechanical stimulation of the breast via movement of the infrasound source's moveable member, expressed milk may fill the chamber relatively quickly. The valve may accordingly beneficially permit release of the expressed milk from the chamber to the milk collection volume, e.g. a milk collection volume that is larger than the volume of the chamber.

In some embodiments, the infrasound souce is arranged to pause the movement, e.g. reciprocating movement, of the moveable member while the expressed milk is being released to the milk collection volume via the valve.

For example, the infrasound source may be arranged to pause the movement, e.g. reciprocating movement, of the moveable member in response to the valve being opened to release the expressed milk to the milk collection volume, and resume the movement of the moveable member in response to the valve being closed to stop release of the expressed milk to the milk collection volume.

For example, a control system, e.g. included in the device or provided separately from the device but in communication with the infrasound source, may control the infrasound source to pause the movement of the moveable member based on the control system receiving an input indicative of the valve being opened.

During milk expression, the chamber's volume becomes progressively filled with milk and hence the chamber's empty volume decreases. This may result in a higher sound pressure inside the chamber.

In some embodiments, the device comprises an acoustic sensor arranged to sense sound pressure in the chamber.

The acoustic sensor may comprise, or be, a microphone.

By the acoustic sensor sensing, e.g. monitoring, the sound pressure, an amount of expressed milk in the chamber can be estimated.

In embodiments in which the device comprises the valve and the acoustic sensor, the valve may be configured to release the expressed milk to the milk collection volume based on the sensed sound pressure, e.g. according to the sensed sound pressure being indicative of the chamber's volume being filled to a given extent.

A controller, e.g. included in the device or provided separately from the device but in communication with the valve, may control the valve to release the expressed milk to the milk collection volume based on the sensed sound pressure.

According to another aspect there is provided a method of operating a device suitable for expressing milk from a mammalian breast, the device comprising a nipple-enclosing wall for interfacing with a nipple-comprising region of the breast to define a chamber between the nipple-enclosing wall and the nipple-comprising region of the breast, an infrasound source for producing infrasound having a moveable member that includes a first side facing and in fluid communication with the chamber, and a second side outside the chamber and opposite the first side, and a fluid connection between the chamber and the second side, the method comprising: decreasing a pressure in the chamber; and causing movement of the moveable member to produce infrasound, the fluid connection controlling fluid communication between the chamber and the second side of the moveable member during the movement of the moveable member.

The method may be a method of operating the device according to any of the embodiments described herein.

The method of operating the device may comprise expressing milk from a non-human mammalian breast.

In embodiments in which the method of operating the device comprises expressing milk, the milk expression may be non-therapeutic milk expression.

Further provided is a computer program comprising computer program code which is configured, when the computer program is run on one or more processors configured to control an infrasound source of a device according to any of the embodiments described herein, to cause the one or more processors to implement the method of operating the device according to embodiments described herein.

In some embodiments, the one or more processors may be further configured to control an underpressure generator arranged to provide the underpressure in the chamber.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
FIG. 1 schematically depicts a device for expressing milk from a mammalian breast;
FIG. 2 schematically depicts another device for expressing milk from a mammalian breast;
FIG. 3 schematically depicts yet another device for expressing milk from a mammalian breast;
FIG. 4 provides a perspective view showing a prototype device for expressing milk from a mammalian breast;
FIG. 5 provides graphs of sound pressure, displacement and power vs infrasound frequency simulated for a device having a chamber with a volume of 28 mL; and
FIG. 6 provides graphs of sound pressure, displacement and power vs infrasound frequency simulated for a device having a chamber with a volume of 14 mL.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Provided is a device for expressing milk from a mammalian breast. The device comprises a nipple-enclosing wall for interfacing with a nipple-comprising region of the breast to define a chamber between the nipple-enclosing wall and the nipple-comprising region of the breast in which an underpressure is providable. The chamber is, for example, connectable to an underpressure generator for decreasing a pressure in the chamber. The underpressure may assist milk to be expressed from the breast and/or may assist in retaining the interface between the nipple-enclosing wall and the nipple-comprising region. The device comprises an infrasound source for producing infrasound. The infrasound source comprises a moveable member whose movement produces infrasound. The moveable member has a first side facing and in fluid communication with the chamber, and a second side opposite the first side. The second side is outside the chamber. A fluid connection is provided between the chamber and the second side. The fluid connection may provide pressure equalization between the chamber and the second side of the moveable member so that both the first side and the second side of the moveable member experience the underpressure. The fluid connection is nonetheless configured to control fluid communication between the chamber and the second side of the moveable member during the movement of the moveable member and thus the fluid connection assists to control, e.g. enhance, a varying pressure experienced by the breast that is caused by the movement of the moveable member.

In other words, the movement of the moveable member, in combination with the fluid connection, enables a varying pressure to be experienced by the breast.

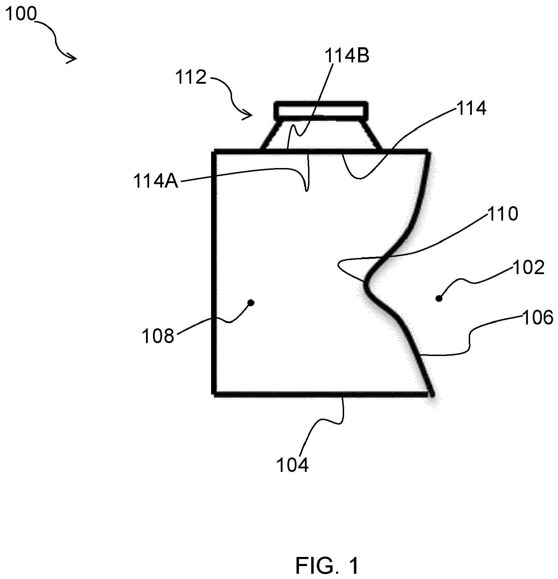

FIG. 1 shows a device 100 for expressing milk from a mammalian breast 102. The device 100 comprises a nipple-enclosing wall 104 for interfacing with a nipple-comprising region 106 of the breast 102. Thus, a chamber 108 is defined between the nipple-enclosing wall 104 and the nipple-comprising region 106 of the breast 102 when the nipple-enclosing wall 104 is interfaced with the nipple-comprising region 106 of the breast 102.

The chamber 108 receives milk from the nipple 110 during milk expression using the device 100.

In at least some embodiments, the nipple-enclosing wall 104 comprises, or is in the form of, a breast shield. Such a breast shield may, for example, be funnel-shaped. Such a funnel-shaped breastshield may assist the nipple-enclosing wall 104 to interface with the nipple-comprising region 106 of the breast 102.

An underpressure is providable in the chamber 108.

For example, the chamber 108 is connectable to an underpressure generator (not visible) for decreasing a pressure in the chamber 108.

Alternatively, such an underpressure may be provided by squeezing a least a portion of the nipple-enclosing wall 104 prior to interfacing the nipple-enclosing wall 104 to the nipple-comprising region 106, and releasing the squeezed nipple-enclosing wall 104 following the interfacing such that a static underpressure is provided in the chamber 108.

More generally, the underpressure pressure provided in the chamber 108 may assist milk to be expressed from the breast 102 and/or may assist in retaining the interface between the nipple-enclosing wall 104 and the nipple-comprising region 106.

The nipple-enclosing wall 104 can be formed of any suitable material provided that the nipple-enclosing wall 104 is capable of interfacing with the nipple-comprising region 106 and enabling an underpressure to be provided in the chamber 108, e.g. by the pressure in the chamber 108 being decreased by the underpressure generator.

In some embodiments, the nipple-enclosing wall 104 is formed from an elastomeric material, such as silicone rubber. Such a material may assist the nipple-enclosing wall 104 to interface with the nipple-comprising region 106 in a way that minimizes ingress of air into the chamber 108 while the pressure therein is decreased.

In at least some embodiments, the underpressure generator is arranged to apply a static underpressure to the chamber 108.

In other words, the underpressure generator may be configured to generate a constant vacuum in the chamber 108.

Such a static underpressure may assist milk to be expressed from the breast 102 and/or may assist in retaining the interface between the nipple-enclosing wall 104 and the nipple-comprising region 106.

In such embodiments, the device 100 can be regarded as a passive milk collector.

The device 100 in the form of such a passive milk collector may, for example, be employed to collect milk from one breast 102 while another breast (not visible) is being used to breastfeed an infant or is being subjected to pumping, in other words active pumping, using a breast pump.

Since the stimulation provided the active pumping can generate a milk ejection reflex on all, e.g. both, breasts, a static underpressure, e.g. generated by the underpressure generator, may be sufficient to extract at least some milk from the breast 102 that is interfaced with the nipple-enclosing wall 104.

In some embodiments, the underpressure generator is configured to provide a pressure, e.g. a static pressure, in the chamber 108 that is lower than atmospheric pressure by at least 80 mBar.

Such an underpressure, in other words a pressure lower than atmospheric pressure, may assist milk extraction from the breast 102.

The design of the underpressure generator is not particularly limited. In some embodiments, the underpressure generator is a manual pump comprising a user-operable actuator that is moveable by the user to decrease the pressure in the chamber 108 when the chamber 108 is connected to the manual pump. Alternatively, the underpressure generator is a powered pump, e.g. a powered pump that is powerable by one or more batteries and/or a mains source of power, configured to decrease the pressure in the chamber 108 when the chamber 108 is connected to the powered pump.

In some embodiments, the device 100 includes the underpressure generator.

Thus, the underpressure generator may be conveniently provided, e.g. supplied, to the user together with the device 100.

Alternatively, the device 100 may not be provided, e.g. supplied, to the user together with the underpressure generator. In such embodiments, the device 100 may, for instance, be connectable to an underpressure generator already in the user's possession.

The device 100 shown in FIG. 1 further comprises an infrasound source 112 for producing infrasound. The infrasound source 112 comprises a moveable member 114, e.g. in the form of a diaphragm, configured to move to produce infrasound.

For example, the moveable member 114 may move in a reciprocating manner in order to produce the infrasound.

Application of the infrasound produced by the infrasound source 112 may assist to stimulate the breast 102, and hence promote expression of milk from the breast 102.

Thus, the infrasound source 112, e.g. an electroacoustic transducer 112, varies the pressure in the chamber 108 in order to provide mechanical stimulation to the breast 102.

As shown in FIG. 1, the moveable member 114 comprises a first side 114A facing and in fluid communication with the chamber 108, and a second side 114B opposite the first side, with the second side 114B being outside the chamber 108.

The first side 114A being in fluid communication with the chamber 108 can be regarded as meaning that the first side 114A is acoustically coupled to the chamber 108.

In at least some embodiments, the infrasound source 112 is configured such that the movement, e.g. reciprocation, of the moveable member 114 is at a frequency in the range of 3 to 20 Hz.

Infrasound in this frequency range has been found to be effective in stimulating the breast 102 to promote expression of milk from the breast 102.

The moveable member 114 can be caused to move in any suitable manner. In some embodiments, the moveable member 114 may be coupled to a piston (not visible) included in the infrasound source 112, with reciprocating movement of the piston causing the moveable member 114 to correspondingly move in a reciprocating manner.

In some embodiments, the infrasound source 112 comprises a linear motor arranged to drive the piston.

In some embodiments, the infrasound source 112 is an electroacoustic transducer comprising a piston in the form of a coil of wire axially displaceable in a magnetic field produced by a magnet, e.g. a permanent magnet. In such embodiments, the reciprocating movement of the moveable member 114, e.g. diaphragm, is provided by induction-driven reciprocation of the coil to which the moveable member 114 is attached.

In some embodiments, the infrasound source 112 comprises, or is in the form of, a loudspeaker.

When the underpressure is provided in the chamber, e.g. by the underpressure generator, and this underpressure is used in combination with the infrasound source 112, the underpressure provided in the chamber 108 can provide a challenge in implementing the infrasound source 112 to promote expression of milk from the breast 102. This may be due to the underpressure, e.g. static vacuum, exerting a force on the infrasound source's piston to introduce an offset from its starting position. Particularly when the pressure in the chamber 108 is lower than atmospheric pressure by 80 mBar or more, the force exerted on the piston may be too high to permit proper actuation of the moveable member 114, e.g. diaphragm.

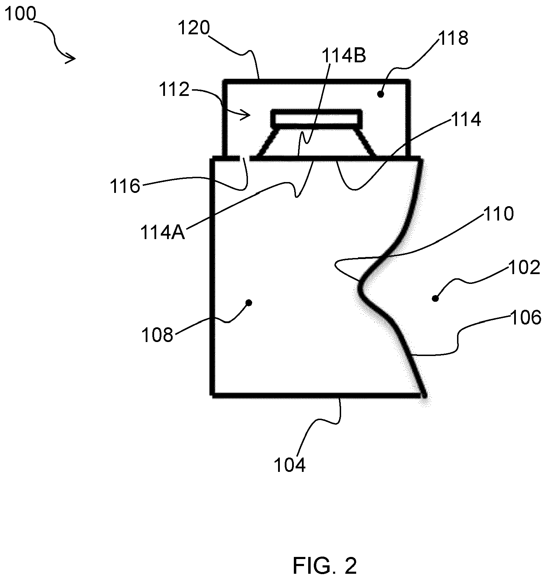

For this reason, in the embodiment shown in FIG. 2 a fluid connection 116 is provided between the chamber 108 and the second side 114B of the moveable member 114.
The fluid connection 116 may provide pressure equalization between the chamber 108 and the second side 114B of the moveable member 114 so that both the first side 114A and the second side 114B of the moveable member 114 experience the underpressure, e.g. the decreased pressure caused by operation of the underpressure generator. This may reduce underpressure-related disruption of movement of the moveable member 114.

In such embodiments, the fluid connection 116 is nonetheless configured to control fluid communication between the chamber 108 and the second side 114B of the moveable member 114 during the movement, e.g. reciprocating movement, of the moveable member 114.

Thus, the fluid connection 116 assists to control, e.g. enhance, a varying pressure experienced by the breast 102 that is caused by the movement of the moveable member 114.

In other words, the infrasound source 112 may vary the pressure in the chamber 108 in order to provide mechanical stimulation to the breast 102 in spite of the fluid connection 116 causing the underpressure to be experienced by both the first side 114A and the second side 114B of the moveable member 114.

In some embodiments, such as that shown in FIG. 2, the fluid connection 116 comprises, or is in the form of, a channel fluidly connecting the chamber 108 to the second side 114B of the moveable member 114.

In such embodiments, the control of the fluid communication between the chamber 108 and the second side 114B of the moveable member 114 may be at least partly determined by the dimensions of the channel.

In other words, the dimensions of the channel may be selected such that the movement of the moveable member 114 causes a varying pressure to be experienced by the breast 102.

For example, the dimensions of the channel can be tuned such that frequencies of pressure variation, for instance 0.5 to 20 Hz, are blocked by the channel so that the pressure variation is experienced by the breast 102, and frequencies of <0.5 Hz are communicated between the chamber 108 and the second side 114B of the moveable member 114.

In some embodiments, the fluid connection 116 is configured to operate as a fluid frequency-dependent resistive element configured to selectively restrict fluid passage between the chamber 108 and the second side 114B of the moveable member 114 driven by a pressure variation at a frequency caused by the movement of the moveable member 114. Thus, the fluid frequency-dependent resistive element may enable the pressure variation caused by the movement of the moveable member 114 to be experienced by the breast 102.

Such a frequency-dependent resistive element may operate due to frequency dependent thermo-viscous losses in the channel included in or defining the fluid connection 116.

In such embodiments, the frequency of the pressure variation caused by the movement of the moveable member 114 may be higher than a frequency of pressure variation resulting from operation of the underpressure generator.

In some embodiments, such as that shown in FIG. 2, the device 100 comprises a further chamber 118, with the second side 114B of the moveable member 114 facing and in fluid communication with the second chamber 118.

In such embodiments, the channel and the further chamber 118 may fluidly connect the chamber 108 to the second side 114B of the moveable member 114.

The further chamber 118 may be defined in any suitable manner. In some embodiments, such as that shown in FIG. 2, the device 100 comprises an infrasound source housing 120 that adjoins the nipple-enclosing wall 104 and extends around the infrasound source 112. In such embodiments, the further chamber 118 may be delimited by the infrasound source housing 120 and an outer portion of the nipple-enclosing wall 104.

In embodiments in which the device 100 includes the further chamber 118, the fluid connection 116 may be configured to operate, together with a volume of the further chamber 118, as an acoustic filter that selectively blocks a frequency of pressure variation caused by the movement of the moveable member 114 from being communicated from the chamber 108 to the further chamber 118. Thus, the acoustic filter may enable the pressure variation caused by the movement of the moveable member 114 to be experienced by the breast 102.

In such embodiments, the frequency of the pressure variation caused by the movement of the moveable member 114 may be higher than a frequency of pressure variation resulting from operation of the underpressure generator.

Hence the acoustic filter may be a low pass filter that allows a lower frequency pressure variation provided by the underpressure generator to be communicated between the chamber 108 and the further chamber 118 but blocks the higher frequency of pressure variation caused by the movement of the moveable member 114 from being communicated from the chamber 108 to the further chamber 118.

The length, L, and area, A, of the channel may be determined by using the well-known Helmholtz resonance frequency, f, defined by f = c/(2^{∗}pi) ^{∗} sqrt(A/(V^{∗}L)), where c is the speed of sound and V is the volume of the further chamber 118.

The Helmholtz resonator assists to avoid pressure waves on each side of the infrasound source's 112 moveable member 114, which are in anti-phase, coming together in the chamber 108 and cancelling each other out, and further assists to minimize the influence of the decreased pressure, e.g. static vacuum, on the neutral position of the moveable member 114, e.g. transducer diaphragm/membrane.

The air inside the channel may act as a mass, and the air inside the volume of the further chamber 118 may act as a spring, forming a mass-spring system. Above the resonance frequency of this mass-spring system, the air may not pass through the channel because of its inertia, while at lower frequencies the inertia may be relatively low, and air can pass through the channel.

Hence, the fluid connection 116 may act as a low pass filter.

The optimal choice of Helmholtz frequency may coincide with the lowest frequency of the pressure variation caused by the movement of the moveable member 114 to be experienced by the breast 102.

The above may, strictly speaking, apply for an ideal system, without damping.

In practice, considering volume, V, and length, L, the channel may be relatively narrow, for example having a diameter in the order of a tenth of a millimeter.

Such narrow channels may have a high amount of thermo-viscous losses (proportional to diameter^4) resulting in a significantly (e.g. up to about 50 times) lower resonance frequency, and thus may act as a more effective low pass filter.

By way of a non-limiting illustrative example/ideal case, the volume, V, of the further chamber 118 is 35 mL, the channel has a length, L, of 5 mm and a diameter, d, of 0.4 mm, giving a Helmholtz resonance frequency of about 20 Hz. When viscous losses are taken into account the resonance frequency lowers to about 0.5 Hz, which is a realistic resonance frequency in the present context.

More generally, the channel may have a diameter, d, of 0.05 to 0.9 mm and/or the channel may have a length of 1 to 10 mm.

In some embodiments, the frequency of pressure variation caused by the movement of the moveable member 114 is equal to or greater than 0.5 Hz, such as 0.5 Hz to 20 Hz.

Alternatively or additionally, the frequency of pressure variation resulting from operation of the underpressure generator may be less than 0.5 Hz.

It is noted that a change in the underpressure in the chamber 108 can still cause an offset in the starting position of the piston of the infrasound source 112 in spite of the inclusion of the fluid connection 116.

In some embodiments, the infrasound source 112 is configured to adjust a starting position of the piston based on the underpressure in the chamber 108.

In this manner, the piston may be adjusted to compensate for a change in the vacuum level during the milk expression. For example, a direct current may be applied, in the case of the infrasound source 112 being an electroacoustic transducer 112, to remove an offset to the starting position caused by a change in the underpressure.

For instance, one or more sensors may be arranged to sense the underpressure pressure, e.g. the static underpressure, in the chamber 108 caused by operation of the underpressure generator and the infrasound source 112 may be configured to adjust the starting position of the piston based on the sensed underpressure.

In some embodiments, the varying pressure experienced by the breast 102 caused by the movement of the moveable member 114 comprises a pressure waveform. Such a pressure waveform may assist to mechanically stimulate the breast 102, and hence promote expression of milk from the breast 102.

Any suitable shape of pressure waveform may be contemplated. In some embodiments, the pressure waveform is a sawtooth waveform, a sinusoidal waveform, or a square waveform.

In some embodiments, the pressure waveform is user-selectable. Thus, the user can choose whichever pressure waveform is perceived as being most effective for them in terms of promoting expression of milk from the breast 102.

In such embodiments, a user interface (not visible) may be configured to permit the user to make a selection of the pressure waveform, e.g. a sawtooth waveform, a sinusoidal waveform, or a square waveform, with the infrasound source 112 being controllable to provide the user-selected pressure waveform.

In some embodiments, the device 100 comprises an air permeable covering (not visible) for restricting liquid passage from the breast 102 to the moveable member 114, e.g. to the first and/or second side 114A, 114B of the moveable member 114.

The air permeable covering may assist to protect the infrasound source 112 from being damaged by the expressed milk in the chamber 108.

Any suitable type of air permeable covering can be contemplated, such as an air permeable covering made from Gore-Tex^{®}.

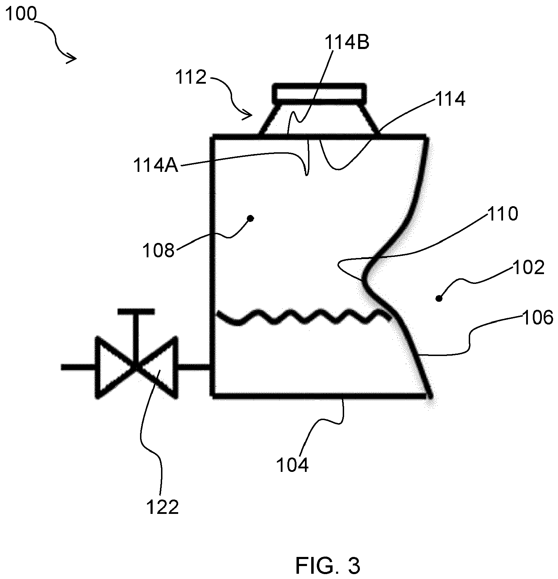

In some embodiments, such as that shown in FIG. 3, the device 100 comprises a valve 122 for selectively releasing the expressed milk from the chamber 108 to a milk collection volume (not visible).

Since it may desirable for the chamber's 108 volume to be kept as small as possible in order to facilitate mechanical stimulation of the breast 102 via movement of the infrasound source's 112 moveable member 114, expressed milk may fill the chamber 108 relatively quickly. The valve 122 may accordingly beneficially permit release of the expressed milk from the chamber 108 to the milk collection volume, e.g. a milk collection volume that is larger than the volume of the chamber 108.

In some embodiments, the milk collection volume is included in the device 100. Alternatively, the milk collection volume may be provided, e.g. supplied, to the user separately from the device 100.

In some embodiments, the infrasound source 112 is arranged to pause the movement of the moveable member 114 while the expressed milk is being released to the milk collection volume via the valve 122.

For example, the infrasound source 112 may be arranged to pause the movement of the moveable member 114 in response to the valve 122 being opened to release the expressed milk to the milk collection volume, and resume the movement of the moveable member 114 in response to the valve 122 being closed to stop release of the expressed milk to the milk collection volume.

In other words, during opening of the valve 122, the mechanical stimulus provided by the infrasound source 112 is temporarily stopped, but following release of the expressed milk, the valve 122 is closed and the mechanical stimulus continues again.

During milk expression, the chamber's 108 volume becomes progressively filled with milk and hence the chamber's 108 empty volume decreases. This may result in a higher sound pressure inside the chamber 108.

In some embodiments, the device 100 comprises an acoustic sensor (not visible) arranged to sense sound pressure in the chamber 108.

The acoustic sensor may comprise, or be, a microphone.

By the acoustic sensor sensing, e.g. monitoring, the sound pressure, an amount of expressed milk in the chamber 108 can be estimated.

In embodiments in which the device 100 comprises the valve 122 and the acoustic sensor, the valve 122 may be configured to release the expressed milk to the milk collection volume based on the sensed sound pressure, e.g. according to the sensed sound pressure being indicative of the chamber's 108 volume being filled to a given extent.

The valve 122 may, for instance, be opened to release the expressed milk to the milk collection volume when the sensed sound pressure is indicative of the chamber's 108 volume being nearly full.

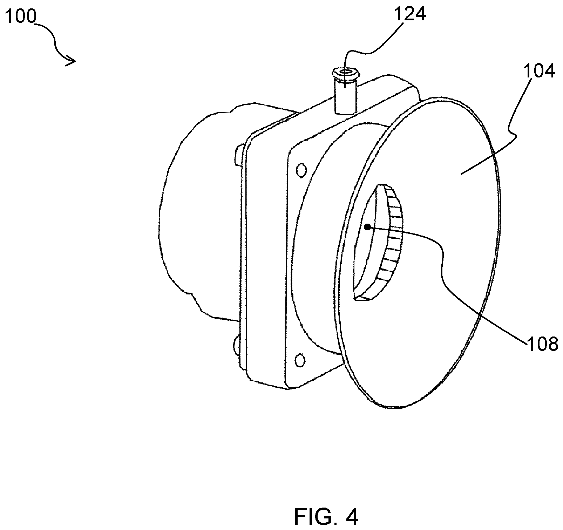

FIG. 4 provides a perspective view showing a prototype of a device 100 for expressing milk from a mammalian breast. The device 100 may have an internal design as depicted in FIG. 2. Whilst the breast 102 is not shown in FIG. 4, the position of the chamber 108 defined between the nipple-enclosing wall 104, e.g. funnel, and where the nipple-comprising region 106 of the breast 102 would be when the nipple-enclosing wall 104 is interfaced thereto is nonetheless denoted in FIG. 4.

In some embodiments, such as that shown in FIG. 4, the device 100 comprises a connection point 124 at which the device 100 is connectable to the underpressure generator (not visible), e.g. via tubing extending between the device 100 and the underpressure generator.

In some embodiments, the device 100 comprises a three-way coupling member (not visible) for connecting the underpressure generator to the chamber 108 and to the further chamber 118.

In existing breast pumps, an amplitude of pressure variation during an extraction period is typically approximately 5000 Pa. A simulation with an electroacoustic simulation toolbox in Matlab^{®} was set up in order to verify that such an amplitude could be realized with a device 100 according to the present disclosure using off-the-shelf components.

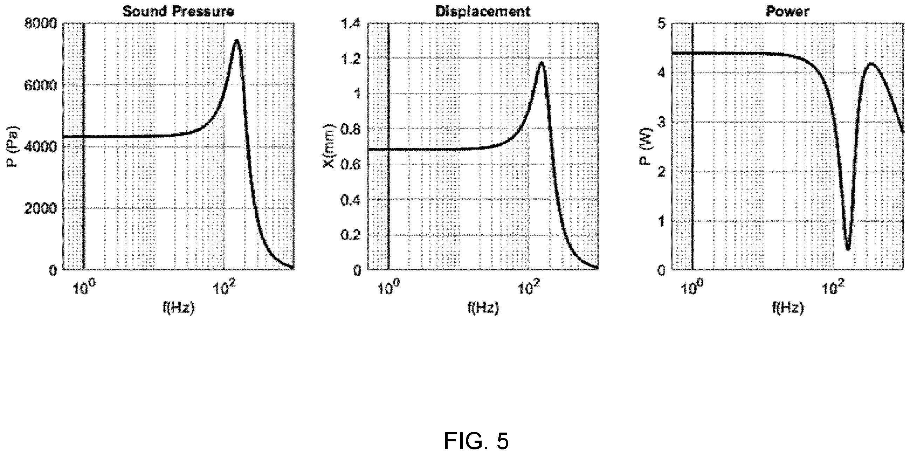

FIG. 5 provides graphs of sound pressure, displacement and power vs infrasound frequency simulated for a device 100 having a chamber 108 with a volume of 28 mL, and a 40 mm infrasound source 112 obtained from a Philips desktop loudspeaker system.

The vertical line in each of the graphs indicates 1 Hz. It is evident from these graphs that the loudspeaker requires approximately 4.5 W with a relatively modest displacement of 0.7 mm to realize an amplitude of 4400 Pa.

Also evident in FIG. 5 is the resonant frequency of the infrasound source 112 at approximately 120 Hz.

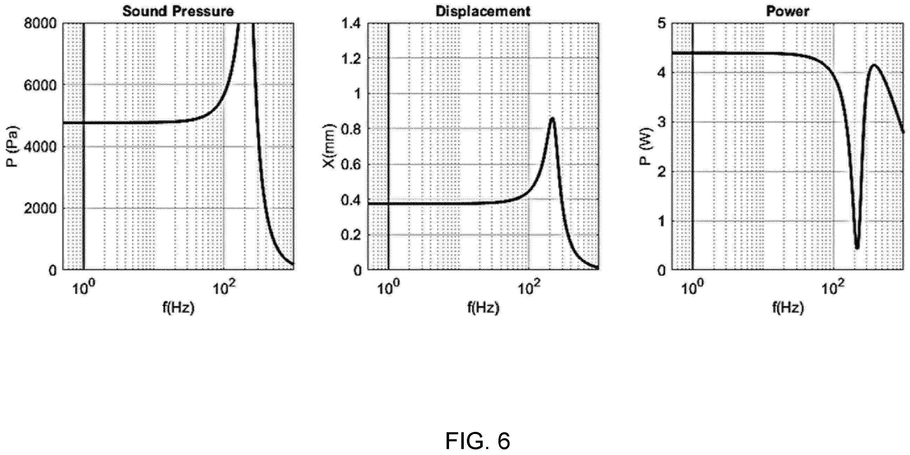

FIG. 6 provides graphs of sound pressure, displacement and power vs infrasound frequency simulated for a device 100 that is the same as that employed for the simulations whose data is graphically presented in FIG. 5 other than the chamber 108 having a volume of 14 mL, rather than 28 mL. Keeping the input power constant at 4.5W, the resulting amplitude increased from 4300 to 4800 Pa, while the required piston displacement reduced from 0.7 to less than 0.4. Also, the resonance frequency shifted to 200 Hz as a consequence of the smaller volume of the chamber 108.

The present disclosure also provides a method of operating a device suitable for expressing milk from a mammalian breast, with the device comprising a nipple-enclosing wall for interfacing with a nipple-comprising region of the breast to define a chamber between the nipple-enclosing wall and the nipple-comprising region of the breast, an infrasound source for producing infrasound having a moveable member that includes a first side facing and in fluid communication with the chamber, and a second side outside the chamber and opposite the first side, and a fluid connection between the chamber and the second side.

The method may be a method of operating the device 100 according to any of the embodiments described herein.

The method comprises decreasing a pressure in the chamber, e.g. by controlling an underpressure generator to decrease the pressure in the chamber, and causing movement of the moveable member.

The fluid connection controls fluid communication between the chamber and the second side of the moveable member during the movement of the moveable member.

The present disclosure further provides is a computer program comprising computer program code which is configured, when the computer program is run on one or more processors configured to control an infrasound source 112 of a device 100 according to any of the embodiments described herein, to cause the one or more processors to implement the method of operating the device 100.

In some embodiments, the one or more processors may be further configured to control an underpressure generator arranged to provide the underpressure in the chamber 108.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A device (100) for expressing milk from a mammalian breast (102), said device comprising:
a nipple-enclosing wall (104) for interfacing with a nipple-comprising region (106) of the breast to define a chamber (108) between the nipple-enclosing wall and the nipple-comprising region of the breast in which an underpressure is providable;
an infrasound source (112) for producing infrasound, the infrasound source comprising a moveable member (114) whose movement produces infrasound, the moveable member having a first side (114A) facing and in fluid communication with the chamber and a second side (114B) opposite the first side, the second side being outside the chamber; and
a fluid connection (116) between the chamber and the second side, the fluid connection being configured to control fluid communication between the chamber and the second side during the movement of the moveable member.

2. The device (100) according to claim 1, wherein the fluid connection (116) comprises a channel fluidly connecting the chamber (108) to the second side (114B) of the moveable member (114), said control of the fluid communication between the chamber and the second side being at least partly determined by the dimensions of the channel.

3. The device (100) according to claim 2, comprising a further chamber (118), the second side (114B) of the moveable member (114) facing and in fluid communication with the second chamber, wherein the channel and the further chamber fluidly connect the chamber (108) to the second side.

4. The device (100) according to claim 3, comprising a three-way coupling member for connecting an underpressure generator to the chamber (108) and to the further chamber (118).

5. The device (100) according to claim 3 or claim 4, wherein the channel is configured to operate, together with a volume of the further chamber (118), as an acoustic filter that selectively blocks a frequency of pressure variation caused by said movement of the moveable member (114) from being communicated from the chamber (108) to the further chamber, thereby to enable said pressure variation to be experienced by the breast (102).

6. The device (100) according to any of claims 1 to 5, wherein the fluid connection (116) is configured to operate as a fluid frequency-dependent resistive element configured to selectively restrict fluid passage between the chamber (108) and the second side (114B) of the moveable member (114) driven by a pressure variation at a frequency caused by said movement of the moveable member, thereby to enable said pressure variation to be experienced by the breast (102).

7. The device (100) according to claim 5 or claim 6, wherein said frequency of pressure variation caused by said movement of the moveable member (114) is equal to or greater than 0.5 Hz.

8. The device (100) according to any one of claims 1 to 7, wherein the infrasound source (112) is configured such that the movement of the moveable member (114) is at a frequency in the range of 3 to 20 Hz.

9. The device (100) according to any one of claims 1 to 8, comprising an air permeable covering for restricting liquid passage from the breast (102) to the moveable member (114), the air permeable covering being arranged to cover the first side (114A) and/or the second side (114B) of the moveable member.

10. The device (100) according to any one of claims 1 to 9, wherein the infrasound source (112) comprises a piston attached to the moveable member (114), movement of the piston causing said movement of the moveable member, and wherein a starting position of the piston is adjustable based on the underpressure in the chamber (108).

11. The device (100) according to any one of claims 1 to 10, wherein said movement of the moveable member (114) provides a pressure waveform, the pressure waveform being user-selectable.

12. The device (100) according to any one of claims 1 to 11, comprising a valve (122) for selectively releasing the expressed milk from the chamber (108) to a milk collection volume.

13. The device (100) according to claim 12, wherein the infrasound source (112) is arranged to be controlled by a control system to pause said movement of the moveable member (114) while the expressed milk is being released to the milk collection volume via the valve.

14. The device (100) according to claim 12 or claim 13, comprising an acoustic sensor arranged to sense sound pressure in the chamber (108), wherein the valve (122) is arranged to be controlled by a controller to release the expressed milk to the milk collection volume based on the sensed sound pressure.

15. A method of operating a device (100) suitable for expressing milk from a mammalian breast (102), the device comprising a nipple-enclosing wall (104) for interfacing with a nipple-comprising region (106) of the breast to define a chamber (108) between the nipple-enclosing wall and the nipple-comprising region of the breast, an infrasound source (112) for producing infrasound having a moveable member (114) that includes a first side (114A) facing and in fluid communication with the chamber and a second side (114B) outside the chamber and opposite the first side, and a fluid connection (116) between the chamber and the second side, the method comprising:
decreasing a pressure in the chamber; and
causing movement of the moveable member to produce infrasound, said fluid connection controlling fluid communication between the chamber and the second side of the moveable member during the movement of the moveable member.
